# EUROPÄISCHE PATENTSCHRIFT

(11) **EP 0 782 993 B1**
(45) Veröffentlichungstag und Bekanntmachung des Hinweises auf die Patenterteilung: **26.02.2003**
(21) Anmeldenummer: 96120823.8
(22) Anmeldetag: 23.12.1996
(51) Int. Cl.: C07D 311/72

(54) **Verfahren zur Herstellung von d,l-alpha-Tocopherol in Gegenwart einer Perfluoralkylensulfonsäure als Katalysator**
Process for the preparation of d,l-alpha-tocopherol in the presence of a perfluoroalkenesulfonic acid catalyst
Procédé pour la production d'alpha-tocophérol en presence d'un catalyseur perfluoroalkylsulfonate

(30) Priorität: 05.01.1996 CH 3196
(43) Veröffentlichungstag der Anmeldung: 09.07.1997
(73) Patentinhaber: F. HOFFMANN-LA ROCHE AG, 4002 Basel (CH)
(72) Erfinder: Bonrath, Werner, 79115 Freiburg (DE)
(74) Vertreter: Kellenberger, Marcus, Dr.

(56) Entgegenhaltungen:
- WO-A-88/02661
- WO-A-95/19222
- DE-A- 2 404 621
- US-A- 3 444 213
- US-A- 3 459 773
- US-A- 3 708 505
- US-A- 4 217 285
- SYNTHESIS, 1986, STUTTGART, Seiten 513-531, XP002029897 OLAH,G.A. ET AL.: "Perfluorinated Resinsulfonic Acid ( Nafion-H) Catalysis in Synthesis"

## Beschreibung

Die vorliegende Erfindung betrifft ein neues Verfahren zur Herstellung von d,l-α-Tocopherol durch Kondensation von Trimethylhydrochinon mit Isophytol. d,l-α-Tocopherol ist ein Diastereomerengemisch eines Derivates der Vitamin E-Gruppe.

Es wurden bereits verschiedene Verfahren zur Herstellung von d,l-α-Tocopherol durch Kondensation von Trimethylhydrochinon mit Isophytol beschrieben.

So wird beispielsweise gemäss Chem. Abstracts (C.A.) **103**, 104799 (1985), C.A. **103**, 123731 (1985) und C.A. **110,** 39221 (1989) die Kondensation in Gegenwart von Zink und Zinkchlorid (ZnCl₂) und einer Protonensäure, wie einer Halogenwasserstoffsäure, z.B. Salzsäure (HCl), Trichloressigsäure, Essigsäure und dergleichen, insbesondere ZnCl₂/HCl, als Katalysator durchgeführt.

Die Herstellung von d,l-α-Tocopherol durch Umsetzung von Trimethylhydrochinon mit Phytylchlorid bzw. Isophytol in Gegenwart von Bortrifluorid (BF₃) oder dessen Etherat (BF₃ · Et₂O) als Katalysator ist in den Deutschen Offenlegungsschriften 960720 und 1015446 sowie in der US-Patentschrift 4,634,781 beschrieben.

Die Kondensation von Trimethylhydrochinon mit Isophytol oder Phytol, das mit Ammoniak oder Aminen behandelt wurde, in Gegenwart von ZnCl₂/HCl oder einer Lewissäure/HCl, wie z.B. BF₃ oder Aluminiumtrichlorid (AlCl₃), als Katalysator ist in der US-Patentschrift 4,634,781 beschrieben.

Gemäss US-Patentschrift 3,789,086 wird die Kondensation in Gegenwart eines FeCl₂/Fe/HCl-Katalysators durchgeführt, während gemäss EP-A 12824 die Kondensation unter Verwendung von Trifluoressigsäure erfolgt .

Alle diese vorbekannten Verfahren weisen gewichtige Nachteile auf: so treten bei allen Verfahren Korrosionsprobleme auf, bei Verwendung von Bortrifluorid zusätzlich noch Toxizitätsprobleme mit den Addukten von Bortrifluorid sowie bei Verwendung von Eisen oder Zink eine heute nicht mehr akzeptable Belastung des Abwassers mit Eisen- oder Zinkionen

Die Verwendung von Ionenaustauschern, z.B Amberlyst 15®, als Katalysator wird in der US-Patentschrift 3 459 773 beschrieben. Das d,l-α-Tocopherol konnte jedoch nach diesem Verfahren nicht in der erforderlichen Reinheit erhalten werden.

Auch die Deutsche Offenlegungsschrift 2 404 621 offenbart die Verwendung eines sauren Katalysators bei der Herstellung von α-Tocopherol durch Kondensation von Trimethylhydrochinon mit Isophytol oder einer verwandten Verbindung. Bei dem Katalysator handelt es sich um eine im Reaktionssystem im wesentlichen unlösliche, feste Säure mit einer maximalen Hammett-Aciditätsfunktion, H₀, von 1,5. Unter den in Frage kommenden sauren Katalysatoren eignen sich Oxide, nicht-flüchtige Metallsalze, nicht-flüchtige Säuren auf Trägern, kristalline Aluminosilikate und natürlich vorkommende Mineralien.

US-Patentschrift 3 708 505 beschreibt die Herstellung von d,l-α-Tocopherol durch die Kondensation von Trimethylhydrochinon mit Isophytol in Gegenwart eines aus einer Lewissäure und mindestens einer starken Säure bestehenden sauren Kondensationsmittels (Katalysators). Als Beispiel des synergistisch wirkenden Katalysators ist u.a. die Kombination von Zinkchlorid und p-Toluolsulfonsäure oder Natriumbisulfat erwähnt.

Gemäss US-Patentschrift 4 217 285 wird die zur Herstellung von dl-α-Tocopherol erfolgende Kondensationsreaktion mit einem zumindest dreiteiligen Katalysatorsystem katalysiert, welches aus Silika-Alumina und/oder Kieselgel, Zinkchlorid und einer Protonensäure, z.B. p-Toluolsulfonsäure oder Salzsäure, besteht.

US-Patentschrift 3 444 213 betrifft eine Methodik zur Herstellung von dl-α-Tocopherol durch die übliche Kondensationsreaktion: Trimethylhydrochinon und Phytol oder ein Derivat davon sowie ein saurer Kondensationskatalysator werden kontinuierlich durch eine geheizte Füllkolonne fliessen gelassen und das gebildete Tocopherol ebenfalls kontinuierlich am Ende der Kolonne entnommen. Geeignete Katalysatoren sind insbesondere Lewissäuren, z.B. Bortrifluorid, Chlorsulfonsäure und p-Toluolsulfonsäure, Fluor- und Bromwasserstoff, sowie ein Gemisch von Fluorwasserstoff und Bortrifluorid.

PCT-Patentpublikation WO 88/02661 offenbart die Verwendung von u.a. Perfluoralkansulfonaten [CF₃(CF₂)ₙSO₃]₃ M (M=B, Al, Ga; n = 0-17) und sogenannten "perfluorinated resinsulfonates", z.B. Nafionate wie Nafion®-H, als festen oder getragenen Katalysatoren für Friedel-Crafts-Reaktionen, z.B. Alkylierung, Isomerisierung, katalytisches Kracken, Polymerisierung, Halogenierung, Acylierung, Formylierung, Sulfonylierung sowie Nitrierung. Eine Anwendung bei der Herstellung von Tocopherol ist nicht angedeutet.

In PCT-Patentpublikation WO 95/19222 ist die Herstellung von porösen Mikrocompositen aus perfluorierten, anhängende Sulfonsäuregruppen und/oder Carbonsäuregruppen enthaltenden Ionenaustauscherpolymeren (PFIEP) mit Metalloxiden durch ein Sol-Gel-Verfahren und die Verwendung der Produkte als Katalysatoren für verschiedene Reaktionen, wie Nitrierung, Veresterung, Polymerisierung, Alkylierung (eines Aromats mit einem Olefin, z.B. Toluol mit n-Hepten), Acylierung sowie α-substituiertes Styren-Dimerisierung, beschrieben. Als Beispiel eines PFIEP ist NAFION® NR 50 genannt. Die Verwendung eines PFIEP oder eines daraus hergestellten porösen Mikrocomposites als Katalysator bei der Herstellung von Tocopherol ist auch in dieser Patentpublikation nicht angedeutet.

Der auf Seiten 513-531 von Synthesis 1986 erscheinende Uebersichtsartikel betreffend "Perfluorinated Resinsulfonic Acid (Nafion-H®) Catalysis in Synthesis" befasst sich u.a. mit der Verwendung solcher Katalysatoren bei der Alkylierung von Aromaten mit Olefinen, z.B. von Benzol mit Ethylen oder Propylen. Wiederum ist die Anwendung bei der Herstellung von Tocopherol nicht angedeutet.

Ein weiterer Artikel, der sich mit der Katalyse mit Nafion befasst, erscheint in der Juli 1987-Ausgabe von Chemtech, auf Seiten 438-441. Neben einer Erläuterung der chemischen Struktur und physikalischen Eigenschaften von Nafion® NR 50, einem Beispiel des PFIEP, ist u.a. in diesem Artikel der Anwendungsbereich offenbart: Katalyse von Acylierungen, Carbonylierungen, Kondensationen (insbesondere 2 C₆H₅NHCO₂R + HCHO → CH₂(C₆H₄NHCO₂R)₂ + H₂O), Bildung von gem-Diacetaten, Umlagerungen, Alkylierungen, Veresterungen, Veretherungen, Hydratisierungen, Nitrierungen sowie Oligomerisierungen. Auch in dieser Literaturstelle ist die oben erwähnte Anwendung allerdings nicht angedeutet. Die Struktur von Nafion® NR 50 ist u.a. als "non-porous" bezeichnet.

In einem noch weiteren Artikel, Chem. and Ind., 18 July 1983, Seiten 568-569, ist die Verwendung des PFIEP-Katalysators Nafion-H bei der von Pechmann-Kondensation eines Phenols, z.B. m-Cresol, mit einer ein aktives Wasserstoffatom aufweisenden Verbindung, z.B. Ethylacetoacetat, zu einem substituierten Coumarin beschrieben und die Vorteile dieses Katalysators gegenüber verschiedenen "konventionellen" sauren Katalysatoren aufgelistet, wie beispielsweise katalytische Menge, leichte Abtrennbarkeit und Wiederverwendung.

Der vorliegenden Erfindung liegt nun die Aufgabe zugrunde, ein Verfahren zur Herstellung von d,l-α-Tocopherol durch Kondensation von Trimethylhydrochinon mit Isophytol in Gegenwart eines Katalysators bereitzustellen, das die Nachteile der vorbekannten Arbeitsweisen nicht aufweist. Dazu ist es notwendig, dass der eingesetzte Katalysator nicht korrosiv wirkt, nicht toxisch ist, die Umwelt nicht belastet, und die erwünschte Reaktion möglichst selektiv und in hohen Ausbeuten katalysiert. Darüber hinaus soll der Katalysator seine Wirkung bereits in wirklich nur katalytischen Mengen entfalten und sich leicht abtrennen und mehrfach wiederverwenden lassen.

Im Rahmen der vorliegenden Erfindung wurde diese Aufgabe dadurch gelöst, dass die Kondensation von Trimethylhydrochinon mit Isophytol in Gegenwart einer Polyperfluoralkylensulfonsäure als Katalysator und in einem aliphatischen oder cyclischen Keton oder einem aliphatischen oder cyclischen Ester als Lösungsmittel durchgeführt wird.

Die Kondensation erfolgt zweckmässigerweise bei Temperaturen zwischen etwa 80°C und 140°C, vorzugsweise zwischen etwa 85°C und 120°C. Zudem werden zweckmässigerweise etwa äquimolare Mengen der beiden Edukte Trimethylhydrochinon und Isophytol eingesetzt.

Als Lösungsmittel werden im Rahmen der vorliegenden Erfindung aliphatische und cyclische Ketone, z.B. Isobutylmethylketon und Diethylketon bzw. Cyclopentanon und Isophoron; und aliphatische und cyclische Ester, z.B. Essigsäure-ethylester, Essigsäure-isopropylester bzw. γ-Butyrolacton, eingesetzt. Bevorzugt sind Diethylketon, Essigsäure-ethylester, Essigsäure-isopropylester und γ-Butyrolacton.

Die Kondensation erfolgt nach folgendem Reaktionsschema:

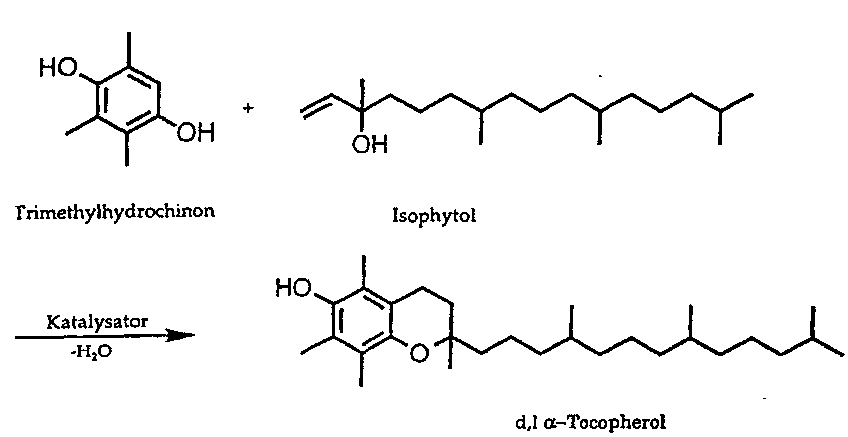

Im erfindungsgemässen Verfahren werden vorzugsweise als saure Kondensationskatalysatoren diejenigen Polyperfluoralkylensulfonsäuren eingesetzt, welche unter dem Markennamen Nafion® erhältlich sind. Ganz bevorzugt sind Nafion NR 50® und Nafion 117®. Die eingesetzten Mengen an Katalysator betragen zweckmässigerweise 1-20 Gew.%, vorzugsweise 1-10 Gew.%, bezogen auf das Gewicht eingesetzten Trimethylhydrochinons oder Isophytols.

Erfindungsgemäss kann zu einer Suspension von Trimethylhydrochinon und Katalysator im Lösungsmittel Isophytol zugetropft werden. Die Zugabegeschwindigkeit an Isophytol ist nicht kritisch. Zweckmässigerweise wird Isophytol in einem Zeitraum von 1-4 Stunden zugetropft. Nach beendeter Isophytolzugabe und entsprechender Nachreaktionszeit erfolgt die Aufarbeitung durch gängige Verfahren der organischen Chemie. Zweckmässigerweise wird die Reaktionslösung vom heterogenen Katalysator durch Filtration oder durch Dekantieren abgetrennt und das Lösungsmittel abgedampft.

Das erfinderische Verfahren bietet die Möglichkeit, den eingesetzten Katalysator leicht abzutrennen und mehrfach wiederzuverwenden.

Die folgenden Beispiele zur Herstellung von d,l-α-Tocopherol durch Kondensation von Trimethylhydrochinon mit Isophytol zeigen vorteilhafte Ausführungsformen des erfindungsgemässen Verfahrens auf, sollen jedoch keinerlei Einschänkung darstellen. Alle Temperaturen sind in Celsiusgraden angegeben.

### Beispiel 1

### Kondensation von Trimethylhydrochinon mit Isophytol in Essigsäureethyester

In einem 500 ml-Vierhalskolben wurden bei Raumtemperatur 31,4 g (200 mmol) Trimethylhydrochinon und 8,5 g des Katalysators Polyperfluoralkylensulfonsäure ( Nafion NR 50 ®) in 100 ml Essigsäure-ethylester suspendiert. Die Suspension wurde auf 85° erwärmt. Innerhalb von 4 Stunden wurden 73 ml (200 mmol) Isophytol zugetropft. Das Lösungsmittel wurde anschliessend abdestilliert.

Ausbeute: 83,55 % der Theorie an d,l-α-Tocopherol.

In einer Variante wurden 9,8 ml Nafion 117® anstelle von 8,5 g Nafion NR 50® zugegeben.

Ausbeute: 63,49 % der Theorie an d,l-α-Tocopherol.

### Beispiel 2

### Kondensation von Trimethylhydrochinon mit Isophytol in Diethylketon

In einem 500 ml-Vierhalskolben wurden bei Raumtemperatur 31,4 g (200 mmol) Trimethylhydrochinon und 15 g des Katalysators Polyperfluoralkylensulfonsäure (Nafion NR 50®) in 50 ml Diethylketon suspendiert. Die Suspension wurde auf 109° erwärmt. Innerhalb von 2 Stunden wurden 73 ml (200 mmol) Isophytol zugetropft. Das Reaktionsgemisch wurde 30 Minuten zum Rückfluss erhitzt. Nach Abkühlen auf Raumtemperatur wurde der Katalysator abfiltriert, und das Filtrat am Rotationsverdampfer eingeengt.

Ausbeute: 84,69 % der Theorie an d,l-α-Tocopherol.

### Beispiel 3

### Kondensation von Trimethylhydrochinon mit Isophytol in γ-Butyrolacton

In einem 500 ml-Vierhalskolben wurden bei Raumtemperatur 31,4 g (200 mmol) Trimethylhydrochinon und 8,5 g des Katalysators Polyperfluoralkylensulfonsäure (Nafion NR 50 ®) in 50 ml γ-Butyrolacton suspendiert. Die Suspension wurde auf 117° erwärmt. Innerhalb von 2 Stunden wurden 73 ml (200 mmol) Isophytol zugetropft. Das Reaktionsgemisch wurde 30 Minuten zum Rückfluss erhitzt. Nach Abkühlen auf Raumtemperatur wurde der Katalysator abfiltriert, und das Filtrat am Rotationsverdampfer eingeengt.

Ausbeute: 84,5 % der Theorie an d,l-α-Tocopherol.

## Patentansprüche

1. Verfahren zur Herstellung von d,l-α-Tocopherol durch Kondensation von Trimethylhydrochinon mit Isophytol, **dadurch gekennzeichnet, dass** die Kondensation in Gegenwart einer Polyperfluoralkylensulfonsäure als Katalysator und in einem aliphatischen oder cyclischen Keton oder einem aliphatischen oder cyclischen Ester als Lösungsmittel durchgeführt wird.

2. Verfahren gemäss Anspruch 1, **dadurch gekennzeichnet, dass** die Kondensation bei Temperaturen zwischen etwa 80°C und 140°C erfolgt.

3. Verfahren gemäss Anspruch 2, **dadurch gekennzeichnet, dass** die Kondensation bei Temperaturen zwischen etwa 85°C und 120°C erfolgt.

4. Verfahren gemäss einem der Ansprüche 1 bis 3, **dadurch gekennzeichnet, dass** als Lösungsmittel Isobutylmethylketon, Diethylketon, Cyclopentanon, Isophoron, Essigsäure-ethylester, Essigsäure-isopropylester oder γ-Butyrolacton eingesetzt wird.

5. Verfahren gemäss Anspruch 4, **dadurch gekennzeichnet, dass** als Lösungsmittel Diethylketon, Essigsäure-ethylester, Essigsäure-isopropylester oder γ-Butyrolacton eingesetzt wird.

6. Verfahren gemäss einem der Ansprüche 1 bis 5, **dadurch gekennzeichnet, dass** 1-20 Gew.% an Polyperfluoralkylensulfonsäure eingesetzt werden.

7. Verfahren gemäss Anspruch 6, **dadurch gekennzeichnet, dass** 1-10 Gew.% an Polyperfluoralkylensulfonsäure eingesetzt werden.

8. Verfahren gemäss einem der Ansprüche 1 bis 7, **dadurch gekennzeichnet, dass** es bei der Polyperfluoralkylensulfonsäure um Nafion NR 50® oder Nafion 117® handelt.

## Claims

1. A process for the manufacture of d,l-α-tocopherol by condensation of trimethylhydroquinone with isophytol, **characterized by** carrying out the condensation in the presence of a polyperfluoroalkylenesulphonic acid as the catalyst and in an aliphatic or cyclic ketone or an aliphatic or cyclic ester as the solvent.

2. A process in accordance with claim 1, **characterized in that** the condensation is carried out at temperatures between about 80°C and 140°C.

3. A process in accordance with claim 2, **characterized in that** the condensation is carried out at temperatures between about 85°C and 120°C.

4. A process in accordance with any one of claims 1 to 3, **characterized in that** isobutyl methyl ketone, diethyl ketone, cyclopentanone, isophorone, ethyl acetate, isopropyl acetate or γ-butyrolactone is used as the solvent.

5. A process in accordance with claim 4, **characterized in that** diethyl ketone, ethyl acetate, isopropyl acetate or γ-butyrolactone is used as the solvent.

6. A process in accordance with any one of claims 1 to 5, **characterized in that** 1-20 wt.% of polyperfluoroalkylenesulphonic acid is used.

7. A process in accordance with claim 6, **characterized in that** 1-10 wt% of polyperfluoroalkylenesulphonic acid is used.

8. A process in accordance with any one of claims 1 to 7, **characterized in that** the polyperfluoroalkylenesulphonic acid is Nafion NR 50® or Nafion 117®.

## Revendications

1. Procédé pour la préparation de d,l-α-tocophérol par condensation de triméthylhydroquinone avec de l'isophytol, **caractérisé en ce que** la condensation est effectuée en présence d'un poly(acide perfluoroalkylènesulfonique) en tant que catalyseur et dans une cétone aliphatique ou cyclique ou un ester aliphatique ou cyclique en tant que solvant.

2. Procédé selon la revendication 1, **caractérisé en ce que** la condensation s'effectue à des températures comprises entre environ 80°C et 140°C.

3. Procédé selon la revendication 2, **caractérisé en ce que** la condensation s'effectue à des températures comprises entre environ 85°C et 120°C.

4. Procédé selon l'une quelconque des revendications 1 à 3, **caractérisé en ce qu'**on utilise comme solvant l'isobutylméthylcétone, la diéthylcétone, la cyclopentanone, l'isophorone, l'acétate d'éthyle, l'acétate d'isopropyle ou la γ-butyrolactone.

5. Procédé selon la revendication 4, **caractérisé en ce qu'**on utilise comme solvant la diéthylcétone, l'acétate d'éthyle, l'acétate d'isopropyle ou la γ-butyrolactone.

6. Procédé selon l'une quelconque des revendications 1 à 5, **caractérisé en ce qu'**on utilise de 1 à 20 % en poids de poly(acide perfluoroalkylènesulfonique).

7. Procédé selon la revendication 6, **caractérisé en ce qu'**on utilise de 1 à 10 % en poids de poly(acide perfluoroalkylènesulfonique).

8. Procédé selon l'une quelconque des revendications 1 à 7, **caractérisé en ce que** le poly(acide perfluoroalkylènesulfonique) consiste en Nafion NR 50® ou Nafion 117®.
